# EUROPÄISCHE PATENTANMELDUNG

(11) **EP 0 732 099 A2**
(43) Veröffentlichungstag der Anmeldung: **18.09.1996**
(21) Anmeldenummer: 96103515.1
(22) Anmeldetag: 07.03.1996
(51) Int. Cl.: A61K 6/083

(54) **Polymerisierbarer Dentalwerkstoff**

(30) Priorität: 13.03.1995 DE 19508586
(71) Anmelder: Degussa Aktiengesellschaft, D-60311 Frankfurt (DE)
(72) Erfinder: Rentsch, Harald, Dr., 63457 Hanau (DE); Mackert, Wilhelm, 63517 Rodenbach (DE)

(57) **Zusammenfassung**

Ein Dentalwerkstoff auf Basis eines organischen polymerisierbaren Bindemittels kennzeichnet sich durch ein ausgewogenes Verhältnis von kugelförmigen auf SiO₂ basierenden Füllstoffen vom Typ A) und asphärischen, splitterförmigen Füllstoffen vom Typ B), wobei die Menge des Füllstoffs A) + B) 1 - 95 Gew.-% beträgt und die kugelförmigen Partikel im auspolymerisierten Dentalwerkstoff separat vorliegen. Füllstoffe vom Typ A) sind A1) reine SiO₂-Partikel mit Brechungsindex < 1,50, A2) mit anorganischen Oxiden beschichtete SiO₂-Partikel und/oder A3) unter A1) oder A2) beschriebene Partikel versehen mit einem Überzug aus einem polymerisierbaren organischen Bindemittel, der bei Einarbeitung in die Matrix auch schon polymerisiert sein kann.

## Beschreibung

Die Erfindung beschreibt einen verbesserten polymerisierbaren Dentalwerkstoff mit variabel einstellbarer Transparenz, guter Polierbarkeit und Festigkeit. Insbesondere bezieht sich die Erfindung auf einen Dentalwerkstoff auf Basis eines polymerisierbaren, ethylenisch ungesättigten Monomeren als Bindemittel, eines Katalysators für die Kalt-, Heiß- und/oder Photopolymerisation und eines anorganischen Füllstoffs, der aus einer Mischung von
A) kugelförmigen auf SiO₂ basierenden Partikeln mit
B) asphärischen, splitterförmigen Pulvern aus Quarz-, Glaskeramik und/oder Glas mit einem Brechungsindex von 1,50 bis 1,58 und einer durchschnittlichen Teilchengröße von 0,5 bis 5,0 µm
besteht.

Zum Stand der Technik werden folgende Druckschriften genannt:
(1) DE-A 32 47 800
(2) DE-A 39 03 407
(3) EP-A 0 238 025
(4) US-A 4,503,169 oder EP-A 0 159 887
(5) EP-A 0 530 926
(6) DE-A 40 29 230

(1) betrifft amorphe, kugelförmige Partikel, Verfahren zu ihrer Herstellung und ihre Verwendung in Dentalmaterialien. Offenbart werden anorganische Verbindungen mit einer Teilchengröße von 0,1 bis 1,0 µm, die als Hauptbestandteile ein Oxid mindestens eines Metalls der Gruppe I, II, III oder IV des Periodensystems, das zur Bindung mit Siliciumdioxid befähigt ist, und Siliciumdioxid enthalten. Die amorphen Partikel werden z. B. aus der Schmelze oder nach einem Sol-Gel-Verfahren hergestellt. Hierbei resultieren Mischoxide, deren Brechungsindices im Bereich von 1,35 bis 1,70 liegen. In den Dentalmaterialien, die einen derartigen Füllstoff ggf. in Mischung mit einem Polymerfüller enthalten, sollen die Partikel nicht agglomerieren, wodurch das Füllungsverhältnis erhöht und die mechanische Festigkeit und die Oberfläche des erhaltenen Dental-Kompositmaterials gesteigert werden. Auch sollen die Transparenz und die Oberflächenglätte der Dentalmaterialien verbessert sein. Obwohl derartige Dentalwerkstoffe hinsichtlich einiger verarbeitungstechnischer Eigenschaften überzeugen können, ist deren mechanische Festigkeit noch verbesserungsbedürftig.
Aus (2) kennt man organisch modifizierte Silikaverbindungen als dentale Füllstoffe. Hierbei handelt es sich um anorganisch-organische Polymere, die als "ORMOCERe" oder "ORMOSILe" bezeichnet werden. Die Herstellung dieser an Silikone erinnernden Polymere erfolgt nach einem Sol-Gel-Prozeß in Anwesenheit saurer oder basischer Katalysatoren. Die ORMOCER-Füllstoffe eignen sich als alleinige Füllstoffe von dentalen Füllmaterialien oder zur Kombination mit anderen Komponenten, wie z. B. silanisiertem Siliciumdioxid, Bor- und Bariumsilikatgläsern, Aluminiumsilikat oder Glaskeramikfüllstoffen. Sie sind durch eine Oberfläche von 10 bis 50 m²/g gekennzeichnet, eine Charakterisierung hinsichtlich Teilchengröße oder Brechungsindex erfolgt nicht. Ein Teil der Siliciumatome der ORMOCER-Struktur kann durch Titan- oder Zirkoniumatome ersetzt werden. Bei ORMOCERen handelt es sich um eine neue Klasse von Verbundwerkstoffen, wobei der Name ORMOCER als Kürzel für ORganically MOdified CERamics ist. Die ORMOCERe bestehen aus atomaren Keramik- und Kunststoff-Netzen, die sich miteinander verbinden und durchdringen.
   Nachteilig beim Einsatz von ORMOCERen oder ORMOSILen in Füllstoffmischungen für Dentalwerkstoffe ist allerdings, daß die beschriebenen ORMOSILe keine transparenten Materialien ergeben, was deren Eignung insgesamt fraglich erscheinen läßt.
(3) hat röntgenopake polymerisierbare Dentalmassen zum Gegenstand, die neben einem oder mehreren ethylenisch ungesättigten polymerisierbaren Monomeren und/oder Polymeren sowie ggf. üblichen Füllstoffen, Pigmenten und weiteren Hilfsmitteln ein schwer lösliches komplexes Schwermetallfluorid enthalten. Es wird darauf hingewiesen, daß die Transparenz der auspolymerisierten Massen stark vom Verhältnis der Brechungsindices der Füllkörper und der polymeren Matrix abhängt, wobei Unterschiede in den Brechungsindices von Polymer und Monomer als gering angesehen werden, wenn sie im Bereich von 1,45 bis 1,6 liegen. Die aus (3) bekannten Füllstoffe liegen alle in Form von asphärischen Splittern vor, was bei Verwendung der in (3) offenbarten Dentalmassen zu einer ungenugenden Polierbarkeit der ausgehärteten Massen führt.
Gemäß (4) sind Composites bekannt, die als Füllstoff bestimmte nicht-gläserne Mikropartikel enthalten, welche im wesentlichen frei von opaken Einschlüssen sind. Hierbei handelt es sich vorzugsweise um SiO₂/ZrO₂-Füllstoffe mit einem Durchmesser < 50 µm. Die Mikropartikel weisen eine Vielzahl amorpher und kristalliner Mikrobereiche auf, wobei die amorphen Bereiche Siliciumdioxid und die kristallinen Bereiche ein radiopakes, keramisches Metalloxid enthalten. Geeignet sind die Oxide von Elementen beispielsweise aus der zweiten bis fünften Hauptgruppe und der dritten bis fünften Nebengruppe des Periodensystems sowie Oxide der Lanthaniden. Bevorzugte keramische Metalloxide sind HfO₂, La₂O₃, SrO und ZrO₂. Die Mikropartikel werden vorzugsweise nach einem Sol-Gel-Verfahren hergestellt. Die Brechungsindices von Füllstoff und Composite-Kunststoff sollten sich bei durch sichtbares Licht härtbaren Zusammensetzungen entsprechen. Abweichungen von 0,05 bzw. 0,005 werden dabei angestrebt. Der Brechungsindex der Mikropartikel läßt sich durch Änderung des Verhältnisses von Siliciumdioxid zum Oxid des keramischen Metalls einstellen. Die in (4) beschriebenen Füllstoffe können mit Mikrofüllstoffen wie z. B. AEROSIL zusammen eingesetzt werden. Aus (4) folgt auf der einen Seite zwar die Wichtigkeit übereinstimmender Brechungsindices von Füllstoff und Composite-Kunststoff, zugleich wird aber in größeren Mengen amorphe Kieselsäure, die bekanntermaßen aggregiert, als sog. Mikrofüller zugesetzt.
(5) offenbart Dentalmassen aus einem polymerisierbaren Monomer und einem anorganischen Füller, der zu 20 bis 80 Gew.-% aus sphärischen anorganischen Oxidpartikeln mit einer durchschnittlichen Teilchengröße zwischen 1,0 und 5,0 µm und zu 80 - 20 Gew.-% aus sphärischen anorganischen Oxidpartikeln mit einer Teilchengröße im Bereich von mindestens 0,05 µm und weniger als 1,0 µm besteht, wobei wenigstens 5 Gew.-% der letztgenannten Komponente im Bereich von 0,05 bis 0,2 µm liegen. Bei den anorganischen Partikeln handelt es sich ausschließlich um kugelförmige Partikel anorganischer Oxide des Siliciums, Zirkoniums, Aluminiums und Titans oder Mischoxide von Metallen aus der I - IV Hauptgruppe des Periodensystems mit Silicium. Die kugelförmigen Partikel werden z. B. durch hydrolytische Polymerisation von Alkoxysilanen hergestellt und können z. B. auch mit γ-Methacryloxypropyltrimethoxysilan oberflächenbehandelt werden.
   Nachteilig an den nur kugelförmige Partikel aufweisenden Füllstoffen ist ebenso wie bei (1) eine unzureichende Festigkeit der resultierenden Dentalwerkstoffe.
Aus (6) ist ein verbesserter Dentalwerkstoff auf Basis eines polymerisierbaren, ethylenisch ungesättigten Monomeren als Bindemittel und eines Katalysators für die Kalt-, Heiß- und/oder Photopolymerisation bekannt, der als anorganischen Füllstoff 20 bis 90 Gew.-% einer Mischung enthält aus
   (A) amorphen, kugelförmigen Teilchen aus Siliciumdioxid und bis zu 20 Mol-% eines Oxids mindestens eines Elements der Gruppen I, II, III und IV des Periodensystems mit einem Brechungsindex von 1,50 bis 1,58 und mit einer durchschnittlichen Primärteilchengröße von 0,1 bis 1,0 um, und
   (B) Quarz-, Glaskeramik- oder Glaspulver oder deren Mischungen mit einem Brechungsindex von 1,50 bis 1,58 und mit einer durchschnittlichen Primärteilchengröße von 0,5 bis 5,0 µm
   sowie ggf. geringe Mengen an weiteren Füllstoffen zur Erhöhung der Opazität und zur Einstellung der Viskosität.

Obwohl die Dentalwerkstoffe gemäß (6) eine gute Transparenz und Polierbarkeit sowie sonstige gute Werkstoffeigenschaften wie Druckfestigkeit, Abriebfestigkeit und Biegefestigkeit aufweisen sollen, hat es sich beim Nacharbeiten herausgestellt, daß die offenbarten SiO₂-Partikel-Mischoxide ein aufwendiges Herstellverfahren erforderlich machen und die Druckfestigkeit des gezeigten Dentalwerkstoffes doch nicht die gestellten Anforderungen erfüllen kann.

In Anbetracht des hierin angegebenen und diskutierten Standes der Technik war es Aufgabe der Erfindung, einen verbesserten Dentalwerkstoff anzugeben, der alle an einen modernen Dentalwerkstoff gestellten Anforderungen hinsichtlich Transparenz, Polierbarkeit, Druckfestigkeit, Wasseraufnahme, Abriebfestigkeit, Biegefestigkeit, Röntgenopazität usw. erfüllt, der leicht herstellbar und an bestimmte spezielle Erfordernisse leicht anpaßbar ist.

Die Lösung dieser Aufgabe gelingt durch einen Dentalwerkstoff der eingangs erwähnten Gattung mit den Merkmalen des kennzeichnenden Teils des Anspruches 1.

Zweckmäßige Weiterbildungen werden in den abhängigen Ansprüchen unter Schutz gestellt.

Dadurch, daß die Menge des Füllstoffs 1 - 95 Gew.-% bezogen auf den Dentalwerkstoff beträgt und daß er als Komponente A) im fertig auspolymerisierten Dentalwerkstoff separat vorliegende kugelförmige Partikel aufweist, aus
A1) SiO₂ mit einem Brechungsindex von zwischen ca. 1,38 und < 1,50 und einer durchschnittlichen Primärteilchengröße von ca. 0,04 µm bis 1,5 µm,
A2) einem SiO₂-Kern, der mit einem Oxid mindestens eines Elements der Gruppen I, II, III und IV des Periodensystems beschichtet ist, wobei die beschichteten Partikel einen Brechungsindex von 1,45 bis 1,62 und eine durchschnittliche Primärteilchengröße von 0,04 bis 1,5 µm aufweisen und die Beschichtung zwischen ca. 15 und 40 nm dick ist, und/oder
A3) unter A1) oder A2) beschriebenen Partikeln, die zusätzlich mit einer Schicht aus einem polymerisierbaren organischen Bindemittel überzogen sind, welches auf mono- oder mehrfachfunktionellen (Meth)acrylaten und/oder Reaktionsprodukten aus Isocyanaten und OH-gruppenhaltigen Methacrylaten beruht, wobei die Primärteilchengröße der mit polymerisierbarer Bindemittelschicht versehenen Partikel zwischen ca. 0,04 und 1,5 µm liegt, während die Schichtdicke der Überzugsschicht im Bereich von 5 nm bis 50 nm und der Brechungsindex der beschichteten Partikel im Bereich von 1,40 - 1,52 liegt,
gelingt es, einen polymerisierbaren Dentalwerkstoff mit variabel einstellbarer Transparenz, guter Polierbarkeit und hoher Festigkeit zu schaffen, bei dem vor allem überraschend war, daß es durch die unter A1) bis A3) beschriebenen Maßnahmen gelang, eine Aggregatbildung der Primärteilchen zu vermeiden. Insbesondere werden durch die erfindungsgemäßen Füllstoffkombinationen von kugelförmigen, separat vorliegenden Teilchen auf SiO₂-Basis und irregulär geformten splitterförmigen Partikeln hohe Raumfüllungsgrade ermöglicht, weil die kleineren kugelförmigen Partikel nicht aggregieren und somit auch die Zwischenräume zwischen den größeren Partikeln maximal gefüllt werden. Dadurch werden diese sog. Zwickel optimal mit Einzelkugeln ausgefüllt, wobei ein aus einer Aggregatbildung resultierender Druckfestigkeitsabfall vermieden wird. Dies war um so überraschender als die kugelförmigen Mischoxid-SiO₂-Primärteilchen im auspolymerisierten Dentalwerkstoff gemäß dem Stand der Technik (Dokument (6)) nicht isoliert vorliegen sondern zu größeren Agglomeraten zusammenbacken, so daß die Primärteilchengröße der Partikel nicht der tatsächlichen Teilchengröße im fertigen Dentalwerkstoff entspricht. Dieses kann z. B. mittels REM-Aufnahmen belegt werden und führt offensichtlich zu verminderten Festigkeitswerten, vor allem der Druckfestigkeit bei Dentalwerkstoffen gemäß dem Stand der Technik.

Dentalwerkstoff bezeichnet im Rahmen der Erfindung Materialien für die Zahnfüllung, Inlays oder Onlays, Zahnzemente, Verblendmaterialien für Kronen und Brücken, Materialien für künstliche Zähne oder sonstige Materialien für die prothetische, konservierende und präventive Zahnheilkunde. Insbesondere fallen unter den Begriff Dentalwerkstoff auch Komposits für zahnmedizinische und zahntechnische Einsatzzwecke, Versieglermaterialien, selbsthärtende Komposits, Stumpfaufbaumaterialien, Verblendkunststoffe, hoch- und normalgefüllte Dualzemente sowie normalgefüllte fluoridhaltige Zahnlacke.

Als Bindemittel kommen für den Dentalwerkstoff alle diejenigen Bindemittel auf Basis eines polymerisierbaren, ethylenisch ungesättigten Monomeren in Frage, die dem Fachmann für diesen Einsatzzweck geläufig sind. Zu den mit Erfolg einsetzbaren polymerisierbaren Monomeren gehören vorzugsweise solche mit acrylischen und/oder methyacrylischen Gruppen.

Insbesondere handelt es sich hierbei u. a. um Ester der α-Cyanoacrylsäure, (Meth)acrylsäure, Urethan(meth)acrylsäure, Crotonsäure, Zimtsäure, Sorbinsäure, Maleinsäure und Itaconsäure mit ein- oder zweiwertigen Alkoholen; (Meth)acrylamide wie z. B. N-isobutylacrylamid; Vinylester von Carbonsäuren wie z. B. Vinylacetat; Vinylether wie z. B. Butylvinylether; Mono-N-vinyl-Verbindungen wie N-Vinylpyrrolidon; und Styrol sowie seine Derivate. Besonders bevorzugt sind die nachfolgend aufgeführten mono- und polyfunktionellen (Meth)acrylsäureester und Urethan(meth)acrylsäureester.
(a) Monofunktionelle (Meth)acrylate
   Methyl(meth)acrylat, N- oder i-Propyl(meth)acrylat, n-, i- oder tert.-Butyl(meth)acrylat und 2-Hydroxyethyl(meth)acrylat.
(b) Difunktionelle (Meth)acrylate
   Verbindungen der allgemeinen Formel: 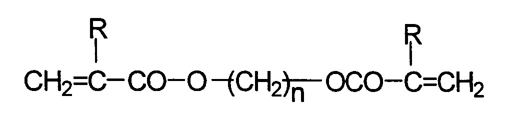 worin R Wasserstoff oder Methyl ist und n eine positive ganze Zahl zwischen 3 und 20, wie z. B. Di(meth)acrylat des Propandiols, Butandiols, Hexandiols, Octandiols, Nonandiols, Decandiols und Eicosandiols, Verbindungen der allgemeinen Formel: 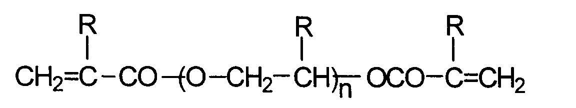 worin R Wasserstoff oder Methyl ist und n eine positive ganze Zahl zwischen 1 und 14, wie z. B. Di(meth)acrylat des Ethylenglycols, Diethylenglycols, Triethylenglycols, Tetraethylenglycols, Dodecaethylenglycols, Tetradecaethylenglycols, Propylenglycols, Dipropylglycols und Tetradecapropylenglycols; und Glycerindi(meth)acrylat, 2,2'-Bis[p-(γ-methacryloxy-β-hydroxypropoxy)-phenylpropan] oder Bis-GMA, Biphenol-A-dimethacrylat, Neopentylglycoldi(meth)acrylat, 2,2'-Di(4-methacryloxypolyethoxyphenyl)propan mit 2 bis 10 Ethoxygruppen pro Molekül und 1,2-Bis(3-methacryloxy-2-hydroxypropoxy)butan.
(c) Tri- oder mehrfachfunktionelle (Meth)acrylate
   Trimethylolpropantri(meth)acrylate und Pentaerythritoltetra(meth)acrylat.
(d) Urethan(meth)acrylat
   Umsetzungsprodukte von 2 Mol hydroxylgruppenhaltigen (Meth)acrylatmonomer mit einem Mol Diisocyanat und Umsetzungsprodukte eines zwei NCO Endgruppen aufweisenden Urethanprepolymers mit einem methacrylischen Monomer, das eine Hydroxylgruppe aufweist, wie sie z. B. durch die allgemeine Formel wiedergegeben werden: 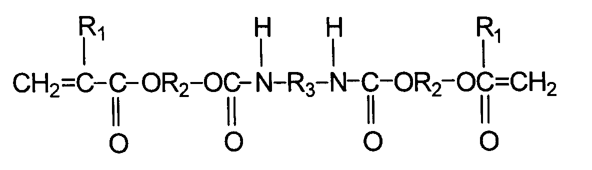 worin R Wasserstoff oder eine Methylgruppe bedeutet, R₂ eine Alkylengruppe und R₃ einen organischen Rest verkörpert.

Die genannten Monomeren werden entweder allein oder in Form einer Mischung von mehreren Monomeren verwendet.

Zu ganz besonders vorteilhaft im erfindungsgemäßen Dentalwerkstoff eingesetzten Monomeren gehören vor allem 2,2-Bis-4(3-methacryloxy-2-hydroxypropoxy)-phenylpropan (Bis-GMA), 3,6-Dioxaoctamethylendimethacrylat (TEDMA) und/oder 7,7,9-Trimethyl-4,13-dioxo-3,14-dioxa-5,12-diazahexadecan-1,16-dioxy-dimethacrylat (UDMA).

Der Dentalwerkstoff kann je nach Art des verwendeten Katalysators heiß, kalt und/oder durch Licht polymersierbar sein. Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis(isobutyroethylester), Benzpinakol und 2,2'-Dimethylbenzpinakol sind geeignet

Als Katalysatoren für die Photopolymerisation können z. B. Benzophenon und seine Derivate sowie Benzoin und seine Derivate verwendet werden. Weitere bevorzugte Photosensibilisatoren sind α-Diketone wie 9,10-Phenanthrenchinon, Diacetyl, Furil, Anisil, 4,4'-Dichlorbenzil und 4,4'-Dialkoxybenzil, Campherchinon wird besonders bevorzugt verwendet. Die Verwendung der Photosensibilisatoren zusammen mit einem Reduktionsmittel wird bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin, N,N-Dimethyl-sym.-xylidin und N,N-3,5-Tetramethylanilin und 4-Dimethylaminobenzoesäureethylester.

Als Katalysatoren für die Kaltpolymerisation werden Radikale liefernde Systeme, z. B. Benzoyl- bzw. Lauroylperoxid zusammen mit Aminen wie N,N-Dimethyl-sym.-xylidin oder N,N-Dimethyl-p-toluidin verwendet. Es können auch dual härtende Systeme zur Katalyse verwendet werden, z. B. Photoinitiatoren mit Aminen und Peroxiden. Als Photokatalysatoren kommen auch Mischungen aus UV-lichthärtenden und im Bereich des sichtbaren Lichts härtenden Katalysatoren in Betracht.

Die Menge dieser Katalysatoren im Dentalwerkstoff liegt üblicherweise zwischen 0,01 bis 5 Gew.-%.

Vorzugsweise dient der erfindungsgemäße Dentalwerkstoff als Zahnfüllungsmaterial. Zahnfüllungsmaterialien werden auch als Zweikonponentenmaterialien hergestellt, die nach dem Anmischen kalt aushärten. Die Zusammensetzung ist ähnlich wie bei den lichthärtenden Materialien, nur wird anstatt der Photokatalysatoren in die eine Paste z. B. Benzoylperoxid und in die andere Paste z. B. N,N-Dimethyl-p-toluidin eingearbeitet. Durch Vermischen etwa gleicher Teile der beiden Pasten erhält man ein Zahnfüllungsmaterial, welches in wenigen Minuten aushärtet.

Wenn man bei den letztgenannten Materialien das Amin wegläßt und als Katalysator z. B. nur Benzoylperoxid verwendet, erhält man einen heißhärtenden Dentalwerkstoff, der für die Herstellung eines Inlays bzw. von künstlichen Zähnen verwendet werden kann. Für die Herstellung eines Inlays wird im Mund des Patienten von der Kavität ein Abdruck genommen und ein Gipsmodell hergestellt. In die Kavität des Gipsmodells wird die Paste eingebracht und das Ganze wird in einem Drucktopf unter Hitze polymerisiert. Das Inlay wird entnommen, bearbeitet und dann im Munde des Patienten in die Kavität einzementiert.

Die erfindungsgemäßen Dentalwerkstoffe verdanken ihre hervorragenden Eigenschaften vorwiegend der Art der verwendeten Füllstoffe A) sowie der wohlausgewogenen Abstimmung des Anteils an kugelförmigen Füllstoffen A) zu splitterförmigen Füllstoffpartikeln B). Dabei ist das Verhältnis A) zu B) u. a. bedeutend für die Festigkeit des auspolymerisierten Dentalwerkstoffes.

Als Füllstoffe A) kommen die Varianten A1) bis A3) oder auch Mischungen dieser Sorten in Frage.

Die Komponenten A1) bis A3) lösen dabei jede für sich allein schon die der Erfindung zugrundeliegende Aufgabe, wobei ihnen insbesondere gemeinsam ist, daß alle Sorten auf SiO₂ basieren oder ausschließlich daraus bestehen und ferner alle Komponenten A1) bis A3) nicht als Agglomerat sondern als separate Partikel im auspolymerisierten Dentalwerkstoff vorliegen.

Erfindungsgemäß können A1) SiO₂-Partikel mit einem Brechungsindex von zwischen 1,38 und < 1,50 mit einer durchschnittlichen Primärteilchengröße von ca. 0,04 µm bis ca. 1,5 µm als Komponente A) eingesetzt werden.

Überraschenderweise konnte bei Verwendung reiner SiO₂-Partikel entgegen der in der Literatur herrschenden Auffassung (vgl. 6) ein Dentalwerkstoff für Füllungen von hervorragender zahnähnlicher Transparenz erhalten werden. Da die Lichtstreuung von der Größe der eingebetteten Partikel abhängig ist, wird bei Verwendung von kleinsten Partikeln, bevorzugt mit Durchmessern im nm-Bereich, die nicht aggregieren, eine Partikelverteilung erreicht, aufgrund derer die Lichtstreuung so gering ist, daß transparente Werkstoffe erhalten werden.

In bevorzugter erfindungsgemäßer Ausführungsform weisen die reinen SiO₂-Partikel einen durchschnittlichen Teilchendurchmesser von ca. 0,04 µm bis ca. 0,25 µm auf. Diese besondere Größe eignet sich vor allem in Dentalwerkstoffen, die speziell bei Füllungswerkstoffen, Verblendwerkstoffen oder künstlichen Zähnen Anwendung finden.

Insbesondere auch für diese Anwendungszwecke ist es besonders zweckmäßig, daß die durchschnittliche Teilchengröße der reinen SiO₂-Partikel insgesamt nicht größer als 0,1 µm ist.

Dort wo die Transparenz des Werkstoffes von untergeordneter Bedeutung ist (beispielsweise bei Stumpfaufbaumaterialien, Zementen für die Befestigung, Versieglern und dgl.), sind auch Partikelgrößen von 0,25 µm bis 1,5 µm bevorzugt. Besonders zweckmäßig liegen die Teilchen dann in einer Größe von 0,25 bis 1,0 µm vor.

Die erfindungsgemäß zu verwendenden reinen SiO₂-Partikel sind bevorzugt monodispers, unporös und essentiell kugelförmig. Grundsätzlich sind alle Oxid-Partikel geeignet, die sich durch hydrolytische Polykondensation aus Alkoholatverbindungen entsprechender Elemente erhalten lassen und dabei in Form monodisperser kompakter sphärischer Partikel anfallen. Die grundliegenden Reaktionsbedingungen zur Herstellung von SiO₂-Partikeln durch hydrolytische Polykondensation sind beispielsweise aus den Publikationen W. Stöber et al. in J. Colloid and Interface Science 26, 62 (1968) und 30, 568 (1969) sowie dem US-Patent 3,634,588 zu entnehmen.

Für die Herstellung von hochmonodispersen, unporösen, kugelförmigen SiO₂-Partikeln, die eine Standardabweichung von nicht mehr als 5 % aufweisen, wird auf EP 0 216 278 hingewiesen, die ein entsprechend abgestelltes Herstellungsverfahren auf Basis von hydrolytischer Polykondensation offenbart. Kern dieses Verfahrens, das für die Herstellung der SiO₂-Partikel gemäß vorliegender Erfindung bevorzugt wird, ist eine zweistufige Vorgehensweise. Hierbei wird zunächst durch hydrolytische Polykondensation von Tetraalkoxysilanen in wässig-alkalisch-ammoniakalischem Medium ein Sol bzw. eine Suspension von Primärteilchen gebildet, die man daran anschließend durch dosierte Zugabe von weiterem Tetralkoxysilan auf die gewünschte Endgröße bringt.

Ein sinngemäßes Verfahren zur Herstellung verschiedener Metalloxide in Form sphärischer Partikel mit enger Teilchengrößenverteilung ist ebenfalls aus EP 0 275 688 zu entnehmen.

Bei den Füllstoffen vom Typ A2) handelt es sich im Rahmen der Erfindung um schichtförmige Partikel, die einen SiO₂-Kern aufweisen, der mit einem Oxid mindestens eines Elementes der Gruppen I, II, III und IV des Periodensystems beschichtet ist.

Hierbei kann das als Kern verwendete SiO₂-Partikel grundsätzlich mit dem unter A1) beschriebenen reinen SiO₂-Partikel identisch sein, i. d. R. weist es jedoch eine um die Dicke der Beschichtung verminderte Teilchengröße als Ausgangsprodukt auf. Es ist wesentlich hervorzuheben, daß die Partikel vom Typ A2) erfindungsgemäß nicht als Mischoxid-Partikel vorliegen, sondern vielmehr der SiO₂-Kern von einem entsprechenden anderen Oxid mindestens eines Elements der Gruppen I bis IV des Periodensystems umgeben ist. Es hat sich überraschenderweise herausgestellt, daß im Gegensatz zu den Mischoxiden die beschichteten Partikel i. d. R. separat und insbesondere nicht aggregiert im auspolymerisierten Dentalwerkstoff vorliegen. Die beschichteten Partikel vom Typ 2 lassen sich grundsätzlich in Analogie zu den unter A1) hierin beschriebenen zweistufigen Verfahren herstellen. Dabei wird bei der hydrolytischen Polykondensation von Tetralkoxysilan in wässig-alkalisch-ammoniakalischem Medium ein Sol bzw. eine Suspension von Primärteilchen gebildet, die man daran anschließend durch dosierte Zugabe eines anderen Tetraalkoxysilans auf die gewünschte Endgröße bringt. So wird also ein Primärpartikel aus einem Oxid gebildet, auf das dann im Aufwachsschritt ein anderes Oxid oder ein Oxidgemisch abgeschieden wird. Hierdurch läßt sich vorteilhaft der resultierende Brechungsindex variieren. Würde die Menge des im Aufwachsschritt gebildeten Oxides in dem fertigen Partikel überwiegen, so wäre es für den resultierenden Brechungsindex im wesentlichen verantwortlich. Zu den besonders bevorzugten auf den SiO₂-Kern abgeschiedenen oxidischen Verbindungen der Gruppen I bis IV des Periodensystemes gehören u. a. TiO₂, ZrO₂, Al₂O₃ und/oder V₂O₅. Dabei ist TiO₂ ganz besonders bevorzugt, wobei bei dessen Verwendung darauf zu achten ist, daß das TiO₂ vollständig gebunden ist, da sonst ggf. Gelbfärbungen des Dentalwerkstoffes auftreten können. Neben den bevorzugten Verbindungen aus den Gruppen I bis IV des Periodensystems sind auch andere Verbindungen möglich. So kann man auch mit Vorteil Nb₂O₅ oder Mischsysteme mit den vorgenannten Oxiden aus den Gruppen I bis IV des Periodensystems mit Erfolg auf SiO₂-Primärpartikeln abscheiden.

Die unter A1) oder A2) beschriebenen Partikel können erfindungsgemäß zusätzlich mit einer Schicht aus einem polymerisierbaren organischen Bindemittel überzogen sein (A3)), welches auf mono- oder mehrfachfunktionellen Methacrylaten und/oder Reaktionsprodukten aus Isocyanaten und OH-Gruppen-haltigen Methacrylaten basiert. Damit ist eine für den jeweiligen Anwendungszweck ggf. vorteilhafte organische Modifizierung der Partikel an der Oberfläche möglich. Diese kann in völliger Übereinstimmung mit Methoden vorgenommen werden, wie sie für die Herstellung für als chromatographische Sorbentien gebräuchlichen Kieselgelen bekannt sind. Gängige Modifizierungsmittel sind dabei Organotrialkoxysilande, wie z. B. Methyltriethoxysilan, Ethyltriethoxysilan, Octyltriethyoxysilan, Octadecyltriethoxysilan, Mono- oder Polyfluoroalkylethoxysilan oder auch Silane mit funktionalisierten Organogruppen, die eine spätere weitere Modifizierung durch kovalente Bindungsknüpfung in bekannter Weise ermöglichen. Im letzteren Fall sind solche Organotrialkoxysilane im Hinblick auf den erfindungsgemäßen Einsatz der Partikel als Füllstoffe in polymeren oder polymerisierbaren Systemen bevorzugt, die solche funktionelle Gruppen aufweisen, mit denen sich eine kovalente Einbindung in das Polymermaterial erreichen läßt. Beispiele hierfür sind Trimethoxyvinylsilan, Triethoxyvinylsilan und 3-Glycidoxypropyltrimethoxysilan, sowie Silane mit Hydroxyl-, Carboxyl-, Epoxy- und Carbonsäureestergruppen tragenden anorganischen Resten. Die Einbindung der solcher Art modifizierten Partikel gemäß A3) in den Dentalwerkstoff geschieht dabei durch Einarbeitung der Partikel in den Dentalwerkstoff und anschließende Polymerisation bei der eigentlichen Aushärtung des Dentalwerkstoffes.

Alternativ hierzu ist es auch möglich, die oberflächenmodifizierten Partikel gemäß A1) oder A2) vor der eigentlichen Einarbeitung in den Dentalwerkstoff zu polymerisieren. Dies kann beispielsweise in Übereinstimmung mit einem Verfahren geschehen, welches im Journal of Colloid and Interface Science 160, 298 - 303 (1993) beschrieben ist. In jedem Falle kann durch geeignete Abstimmung des zur Oberflächen-Modifizierung verwendeten Monomeren auf das Monomer oder die Monomermischung, die die Polymermatrix des Dentalwerkstoffes ausbildet, eine Feinabstufung und Regulierung des Brechungsindex des gesamten Dentalwerkstoffes erzielt werden.

Bei dem anorganischen Füllstoff (B) des Füllstoffgemisches handelt es sich um Quarz-, Glaskeramik- oder Glaspulver. Bevorzugt werden Gläser verwendet. Die durchschnittliche Primärteilchengröße des anorganischen Füllstoffes (B) soll zwischen 0,5 und 5,0 µm , insbesondere zwischen 1,0 und 2,0 µm und besonders bevorzugt zwischen 1,0 und 1,5 µm liegen, während der Brechungsindex Werte zwischen 1,50 und 1,58, insbesondere zwischen 1,52 und 1,56 aufweisen soll. Es können auch Füllstoffgemisches eingesetzt werden.

Bevorzugt werden erfindungsgemäß Ba-Silikatgläser mit einer mittleren Korngröße im Bereich von 1,1 bis 1,3 µm, sowie Sr-Silikatgläser mit einer mittleren Korngröße im Bereich von 1,1 bis 1,3 µm, sowie Li/Al-Silikatgläser mit einer mittleren Korngröße von 1,0 bis 1,6 µm verwendet. Solche Pulver lassen sich z. B. durch Feinmahlung mit einer herkömmlichen Ultrafeinstmühle erhalten.

Als splitterförmige Füllstoffe vom Typ (B) kommen auch Polysiloxan-Füllstoffe in Frage, die Aluminium-haltig sind und aus Einheiten der Formel 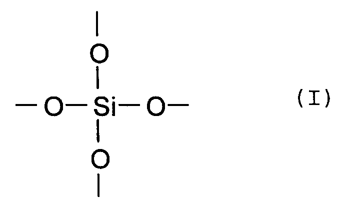 und Einheiten der Formel 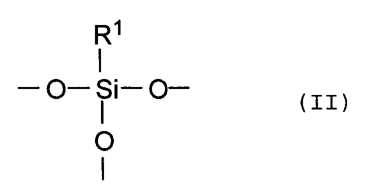 wobei R¹ für eine mit einem Acrylat- oder Methacrylatrest verbundene lineare oder verzweigte Alkylgruppe mit 1 bis 6 C-Atomen oder für einen einfach olefinisch ungesättigten linearen oder verzweigten Kohlenwasserstoffrest mit 2 bis 8 C-Atomen oder für einen zyklischen, einfach olefinisch ungesättigten Kohlenwasserstoffrest mit 5 - 8 C-Atomen oder für eine lineare oder verzweigte Alkylgruppe mit 1 bis 8 C-Atomen, eine Cycloalkylengruppe mit 5 bis 8 C-Atomen, eine Phenylgruppe odere ine Alkylarylgruppe steht, und/oder Einheiten der Formel 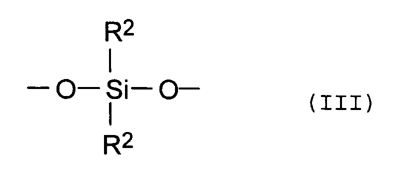 in denen R² eine Methyl-, Ethyl-, Propyl- oder Phenylgruppe repräsentiert, besteht und - bei jeder der Zusammensetzungen - Einheiten der Formel 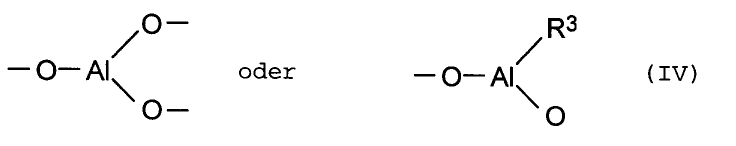 in denen R³ eine lineare oder verzweigte Alkylgruppe mit 1 bis 5 C-Atomen oder eine Phenylgruppe ist, vorliegen und die freien Valenzen der an die Silicium- und Aluminiumatome gebundenen Sauerstoffatome bei den Einheiten (I), (II) und/oder (III) sowie (IV) wie bei Heterosiloxangerüsten durch ein Siliciumatom einer gleichen oder einer unterschiedlichen Einheit oder durch ein Aluminiumatom abgesättigt werden, wobei das Verhältnis der Siliciumatome aus den Einheiten der Formel (I) zu der Summe der Siliciumatome der Einheiten (II) und (III) 3 : 1 bis 100 : 1 und das Verhältnis der Summe der Siliciumatome aus den Einheiten (I), (II) und (III) zu den Aluminiumatomen aus den Einheiten (IV) 2 : 1 bis 200 : 1 beträgt.

Dabei kann das Aluminum im Polysiloxan-Füllstoff ggf. auch durch ein anderes Metall ersetzt sein.

Gegebenenfalls können noch weitere Füllstoffe (C) zur Erzielung einer erhöhten Röntgenopazität eingesetzt werden, wobei deren mittlere Primärteilchengröße 5,0 µm nicht übersteigen sollte. Solche Füllstoffe sind z. B. in der DE-OS 35 02 594 beschrieben.

Gegebenenfalls können zur Einstellung der Viskosität geringe Mengen an mikrofeiner, pyrogener oder naßgefällter Kieselsäure (Füllstoff (D)) in den Dentalwerkstoff eingearbeitet werden, höchstens jedoch 5 Gew.-%, bezogen auf den Dentalwerkstoff.

In bevorzugter erfindungsgemäßer Ausführungsform kennzeichnet sich der Dentalwerkstoff dadurch, daß der Gewichtsanteil an Füllstoff (A) 1 - 60 Gew.-% und an Füllstoff (B) 15 - 85 Gew.-% beträgt, wobei beide Gew.-%-Angaben jeweils auf das Gesamtgewicht des Dentalwerkstoffes bezogen sind. Durch eine Wahl der Mengen an Füllstoffen in diesem Bereich gelingt es besonders vorteilhaft, das Verhältnis von splitterförmigen Bestandteilen vom Typ B zu kugelförmigen separat vorliegenden Partikeln vom Typ A zu optimieren.

Weiterhin zweckmäßig ist es, das Verhältnis von Füllstoff A) zu Füllstoff B) so im fertigen Dentalwerkstoff einzustellen, daß eine Aggregatbildung der Füllstoffe (A) vermieden wird. Dies ist vom Fachmann durch entsprechende Versuche empirisch zu ermitteln.

In besonders bevorzugter Ausführungsform wird das Verhältnis der Füllstoffkomponenten (A) zu den Füllstoffen (B) im Bereich von 1 : 85 bis 4 : 1 (jeweils bezogen auf Gew.-%) so eingestellt, daß die Festigkeit des resultierenden Dentalwerkstoffes bezogen auf seine Druckfestigkeit im Bereich von > 320 bis ca. 480 MPa beträgt. Hierbei hat es sich als besonders günstig herausgestellt, wenn das Verhältnis von Füllstoffen A) zu Füllstoffen B) ≧ 1 : 10 beträgt (jeweils bezogen auf Gew.-%).

Nachfolgend wird die Erfindung anhand von Ausführungsbeispielen und Vergleichsbeispielen weitergehend erläutert.

### Beispiele

Zur Herstellung der nachfolgend beschriebenen Pasten wurde ein handelsüblicher Kneter der Fa. Grieser verwendet. Seine Knetwerkzeuge wurden so modifiziert, daß eine besonders intensive Durchmischung und homogene Verteilung der Ausgangsstoffe möglich war. Die Homogenisierung der Monomermischungen erfolgte üblicherweise über einen Dreiwalzenstuhl.

Bei lichthärtenden Systemen betrug die Aushärtungszeit 40 sec mit einer handelsüblichen Lampe (Degulux® der Firma Degussa).

### Methodenbeschreibung:

### Transparenz:

Die Bestimmung der Transparenz erfolgt an Probenkörpern der Dicke d=3±0,1 mm und einem Durchmesser von 20±0,1 mm. Zur Herstellung der Proben wird die in die Stahlformen gleicher Dimensionen eingefüllte Kompositpaste mit 4000 kp für 30 sec belastet und anschließend für 2 min mit einer Dentallampe der Lichtintensität von mindestens 250 rel. Einheiten ausgehärtet. Während des Aushärtungsvorgangs ist die Kompositoberfläche durch eine transparente Polyesterfolie vom Luftsauerstoff abgeschirmt. Die Messung der Transparenz erfolgt mit einem UV/VIS Spektrophotometer PU8800 (Philips) in Transmissionsmodus.

### Druckfestigkeit:

Das Komposit wird blasenfrei in ein Duranglasrohr der Höhe h=9 mm und einem Durchmesser d=4mm und 40 sec lang mit einer Dentallampe der Lichtintensität von mindestens 250 rel. Einheiten ausgehärtet. Nach dem Entformen wird der Prüfkörper mit einer Diamantsäge auf 6,0 mm verkürzt und für 24h in destilliertem Wasser bei 37 Grad Celsius gelagert. Die Messung der Druckfestigkeit erfolgt mit einer Universalprüfmaschine der Fa. Frank. Den Zahlenwerten liegen Mittelwerte aus 7 Einzelmessungen zugrunde.

In den nachfolgenden Beispielen werden die Füllstoffe A1, A2 und A3 wie folgt verwendet: A1: amorphe SiO₂-Partikel mit einem Durchmesser von 80-1000 nm, Brechungsindex ca. 1,4; A2: SiO₂-Kern von 500nm + 30nm dicke Schicht aus TiO₂, Brechungsindex 1,49; A3: SiO-Kern von 500nm + 50nm dicke Schicht aus Bis-GMA, Brechungsindex 1,47.

### Beispiel 1:

In 22,86g einer Monomermischung, bestehend aus 45 Teilen Bis-GMA, 20 Teilen UDMA und 35 Teilen TEDMA, werden 71,14g silanisiertes Barium-Silikatglas, 5g Aerosil und 1g des kugelförmigen, monodispersen und nichtaggregiert im Werkstoff vorliegenden Füllstoffes A1, Partikeldurchmesser 500 nm zusammen mit 0,038 Gew.% Campherchinon eingearbeitet. Die entstandene Paste wurde mit Licht (Degulux® )ausgehärtet.
Transparenz: 21,7%

### Beispiel 2:

wie Beispiel 1, aber der Partikeldurchmesser des Füllstoffes A1 beträgt 100 nm.
Transparenz: 33,4%

### Beispiel 3:

In 21g einer Monomermischung analog Beispiel 1 werden 65g silanisiertes Barium-Silikatglas, 5g Aerosil und 9g des monodispersen Füllstoffes A1, Partikeldurchmesser 100 nm zusammen mit 0,038 Gew.% Campherchinon eingearbeitet. Die entstandene Paste wurde mit Licht (Degulux® ) ausgehärtet.
Transparenz: 20,3%

### Beispiel 4:

wie Beispiel 3, aber der Partikeldurchmesser des Füllstoffes A1 beträgt 80 nm.
Transparenz: 24,1%

### Beispiel 5:

In 18,2g einer Monomermischung, bestehend aus 20 Teilen Bis-GMA, 55 Teilen UDMA und 25 Teilen TEDMA werden 64 g silanisiertes Ba-Silikatglas, 10,7g silanisiertes Aerosil und 7,1g des Füllstoffes A1 mit einem Durchmesser von 1000 nm zusammen mit 0,038 Gew.% Campherchinon eingearbeitet. Es entsteht ein geschmeidiges Material.
Transparenz: 11,9%

### Beispiel 6:

In 21g einer Monomermischung analog Beispiel 1 werden 69g silanisiertes Barium-Silikatglas, 5g Aerosil und 5g des kugelförmigen, monodispersen und nicht aggregierten Füllstoffes A1, Partikeldurchmesser 95 nm zusammen mit 0,038 Gew.% Campherchinon eingearbeitet. Die Paste erhält nach Zugabe von Pigmenten ein zahnfarbenes Aussehen. Die entstandene Paste wurde mit Licht (Degulux®) ausgehärtet. Der erhaltene Werkstoff eignet sich als lichthärtendes, zahnfarbenes Füllungsmaterial für Front- und Seitenzahngebiet sowie als Material für Inlays und Onlays.
Transparenz: 23,0%
Druckfestigkeit: 420 MPa

### Vergleichsbeispiel 7:

In 17,3g einer Monomermischung, bestehend aus 42,2 Teilen Bis-GMA, 36,4 Teilen UDMA und 21,4 Teilen TEDMA werden 46,8 g Barium-Silikatglas, 3,8g Aerosil, 15,4g Ytterbium(III)-fluorid und 16,5 g kugelförmiger Füllstoff nach DE 4029230 A1 eingearbeitet. Zusätzlich wurden geringe Mengen Farbpigment zugegeben
Transparenz: 21,0%
Druckfestigkeit: 300 MPa

### Beispiel 8:

### Basispaste:

In 22,4g einer Monomermischung, bestehend aus 54,9 Teilen UDMA, 20,1 Teilen Bis-GMA und 25 Teilen TEDMA werden 59,5g silanisiertes Barium-Silikatglas, 7,2g Aerosil, 0,5g Aktivator/Stabilisator und 10,4g Füllstoff A1 oder A2 oder A3 oder Kombinationen derselben eingearbeitet. Nach Zugabe einer kleinen Menge Pigmente entsteht eine hochviskose, zahnfarbene Paste

### Katalysatorpaste:

In 22,4g einer Monomermischung, bestehend aus 54,9 Teilen UDMA, 20,1 Teilen Bis-GMA und 25 Teilen TEDMA werden 59,5g silanisiertes Barium-Silikatglas, 7,2g Aerosil, 0,5g Benzoylperoxid und 10,4g Füllstoff A1 oder A2 oder A3 oder Kombinationen derselben eingearbeitet. Nach Zugabe einer kleinen Menge Pigmente entsteht eine hochviskose, zahnfarbene Paste, welche nach Mischen mit gleichen Teilen Basispaste als selbsthärtendes Füllungsmaterial oder zur Herstellung von heißhärtenden Inlays geeignet ist.
Transparenz: 21,2%

### Beispiel 9:

### Basispaste:

In 30g einer Monomermischung, bestehend aus 28,9 Teilen TEDMA und 71,1 Teilen UDMA werden 59g silanisiertes Barium-Silikatglas, 6g des kugelförmigen Füllstoffes A1 und 5g silanisiertes Aerosil zusammen mit 1,15% eines Aktivator/Stabilisisatorgemisches und 0,09% Champherchinon eingearbeitet. Nach der Zugabe kleiner Mengen von Pigmenten entsteht eine zahnfarbene, zähfließende Paste.

### Katalysatorpaste:

In 30g einer Monomermischung, bestehend aus 28,9 Teilen TEDMA und 71,1 Teilen UDMA werden 59g silanisiertes Barium-Silikatglas, 6g des kugelförmigen Füllstoffes A1 und 5g silanisiertes Aerosil zusammen mit 0,6% Benzoylperoxid eingearbeitet. Nach der Zugabe kleiner Mengen von Pigmenten entsteht eine zahnfarbene, zähfließende Paste.

Nach dem Vermischen beider Pasten zu entsteht eine licht- und selbsthärtende Dental-Zementpaste.
Transparenz: 22,0%

### Beispiel 10:

In 96g einer Monomermischung, bestehend aus 60 Teilen Bis-GMA und 40 Teilen TEDMA werden 3g des kugelförmigen, monodispersen und separat im Werkstoff vorliegenden Füllstoffes A1 und 1g Titandioxid zusammen mit 0,12 % Campherchinon eingearbeitet. Es entsteht ein weißes dünnfließendes Material, welches nach einem Jahr Lagerung keine Sedimentation der Füllstoffe aufweist. Der erhaltene Dentalwerkstoff eignet sich zur Versieglung von Fissuren und Zahnoberflächen.
Transparenz: 30,3% (Schichtdicke=0,5mm)

## Patentansprüche

1. Dentalwerkstoff auf Basis eines polymerisierbaren, ethylenisch ungesättigten Monomeren als Bindemittel, eines Katalysators für die Kalt-, Heiß- und/oder Photopolymerisation und eines anorganischen Füllstoffs, der aus einer Mischung von
A) kugelförmigen auf SiO₂ basierenden Partikeln mit
B) asphärischen, splitterförmigen Pulvern aus Quarz-, Glaskeramik und/oder Glas mit einem Brechungsindex von 1,50 bis 1,58 und einer durchschnittlichen Teilchengröße von 0,5 bis 5,0 µm
besteht,
**dadurch gekennzeichnet,**
daß die Menge des Füllstoffs 1 - 95 Gew.-% bezogen auf den Dentalwerkstoff beträgt und daß er als Komponente A) im fertig auspolymerisierten Dentalwerkstoff separat vorliegende kugelförmige Partikel aufweist, aus
A1) SiO₂ mit einem Brechungsindex von zwischen ca. 1,38 und < 1,50 und einer durchschnittlichen Primärteilchengröße von ca. 0,04 µm bis 1,5 µm,
A2) einem SiO₂-Kern, der mit einem Oxid mindestens eines Elements der Gruppen I, II, III und IV des Periodensystems beschichtet ist, wobei die beschichteten Partikel einen Brechungsindex von 1,45 bis 1,62 und eine durchschnittliche Primärteilchengröße von 0,04 bis 1,5 µm aufweisen und die Beschichtung zwischen ca. 15 und 40 nm dick ist, und/oder
A3) unter A1) oder A2) beschriebenen Partikeln, die zusätzlich mit einer Schicht aus einem polymerisierbaren organischen Bindemittel überzogen sind, welches auf mono- oder mehrfachfunktionellen (Meth)acrylaten und/oder Reaktionsprodukten aus Isocyanaten und OH-gruppenhaltigen Methacrylaten beruht, wobei die Primärteilchengröße der mit polymerisierbarer Bindemittelschicht versehenen Partikel zwischen ca. 0,04 und 1,5 µm liegt, während die Schichtdicke der Überzugsschicht im Bereich von 5 nm bis 50 nm und der Brechungsindex der beschichteten Partikel im Bereich von 1,40 - 1,52 liegt.

2. Dentalwerkstoff nach Anspruch 1,
**dadurch gekennzeichnet,**
daß der Gewichtsanteil an Füllstoff (A) 1 bis 60 Gew.-% und an Füllstoff (B) 15 bis 85 Gew.-%, jeweils bezogen auf den Dentalwerkstoff, beträgt.

3. Dentalwerkstoff nach Anspruch 1 oder 2,
**dadurch gekennzeichnet,**
daß das Verhältnis von Füllstoff (A) zu Füllstoff (B) so eingestellt ist, daß eine Aggregatbildung der Füllstoffe (A) vermieden wird.

4. Dentalwerkstoff nach einem der vorhergehenden Ansprüche,
**dadurch gekennzeichnet,**
daß das Verhältnis von (A) zu (B) im Bereich von 1 : 85 bis 4 : 1 so eingestellt wird, daß die Festigkeit des Dentalwerkstoffes bezogen auf seine Druckfestigkeit von > 320 bis 480 MPa beträgt.

5. Dentalwerkstoff nach einem der vorhergehenden Ansprüche,
**dadurch gekennzeichnet,**
daß das Verhältnis von (A) zu (B) ≧ 1 : 10 beträgt.
